# FASCICULE DE BREVET EUROPEEN

(11) **EP 3 122 186 B1**
(45) Date de publication et mention de la délivrance du brevet: **25.03.2020**
(21) Numéro de dépôt: 15719263.4
(22) Date de dépôt: 24.03.2015
(51) Int. Cl.: A01N 37/02, A01P 7/04, A01N 25/04

(54) **UTILISATION D'ESTER(S) D'ACIDE(S) GRAS COMME INSECTICIDE**
VERWENDUNG VON FETTSÄUREESTER ALS INSEKTIZID
USE OF FATTY ACID ESTER(S) AS INSECTICIDE

(30) Priorité: 26.03.2014 FR 1452607
(43) Date de publication de la demande: 01.02.2017
(73) Titulaire: OLEON NV, 9940 Evergem (Ertvelde) (BE)
(72) Inventeur: RAVIER, Pierre, F-60200 Compiegne (FR); CHATILLON, Matthieu, F-60200 Compiegne (FR); BARREAU, Sébastien, F-60400 Noyon (FR)
(74) Mandataire: Santarelli
(86) Numéro de dépôt international: PCT/FR2015/050734
(87) Numéro de publication internationale: WO 2015/145057

(56) Documents cités:
- WO-A1-96/22020
- DE-A1- 4 445 546
- FR-A1- 2 377 154
- FR-A1- 2 526 632
- US-A1- 2012 121 737
- US-A1- 2013 203 842

## Description

La présente invention se rapporte à la lutte contre les insectes. Elle vise plus particulièrement l'utilisation de certains esters d'acide(s) gras et/ou leur mélange comme insecticide et des compositions (concentrés, solutions) insecticides les comportant. La présente invention concerne également un procédé de lutte contre les insectes.

Les insecticides et/ou les arachnicides sont des produits pharmaceutiques ou phytopharmaceutiques destinés à lutter contre les insectes et/ou les arachnides respectivement. Il existe aujourd'hui de nombreux insecticides et arachnicides, classés par familles chimiques, celles-ci regroupant les insecticides et/ou arachnicides présentant un mode d'action similaire. Toutefois, de par leur mode d'action, la plupart des composés insecticides et/ou arachnicides aujourd'hui disponibles a un impact non négligeable sur l'environnement et/ou la santé.

A titre d'exemple, les composés organochlorés présentent l'avantage d'être faiblement toxique pour l'homme. Toutefois, leur grande stabilité et leur biodégradabilité en métabolite(s) encore plus stable(s) ont causé des problèmes d'accumulation dans les organismes et les écosystèmes, amenant certains pays à interdire leur utilisation.

A l'inverse, les composés organophosphorés, très utilisés aujourd'hui, présentent une forte toxicité mais une bonne biodégradabilité.

Les pyréthrinoïdes, quant à eux, présentent une faible toxicité pour l'homme et sont très biodégradables. Cependant, ils semblent être toxiques pour certains organismes aquatiques et pour les auxiliaires de l'agriculture (tels que les abeilles). De plus, certains insectes, tels que les méligèthes, ont développé une résistance à l'égard des pyréthrinoïdes.

En effet, l'IRAC (Insecticide Résistance Action Committee) a publié en janvier 2013 le résultat d'une étude sur la résistance des méligèthes aux pyréthrinoïdes. Il ressort de cette étude que seule 7% de la population des méligèthes reste susceptible aux pyréthrinoïdes.

En outre, ce phénomène de résistance aux insecticides et/ou aux arachnicides n'est pas un phénomène limité aux méligèthes et aux pyréthrinoïdes mais est au contraire un phénomène en plein développement qui touche de nombreux types d'insectes et/ou arachnides et différents types d'insecticides et/ou d'arachnicides. Ce phénomène de résistance, développé par les différents insectes et/ou arachnides, est de plus accentué par l'application répétitive d'insecticides et/ou d'arachnicides présentant un même mode d'action. Afin d'éviter ce phénomène de résistance, les stratégies d'application des insecticides et/ou arachnicides se sont concentrées vers une alternance des modes d'action et plus récemment, mais de manière restreinte, sur une combinaison de ceux-ci.

Il résulte de ce qui précède qu'un besoin existe pour de nouveaux insecticides/arachnicides et/ou combinaisons d'insecticides et/ou d'arachnicides ayant un impact réduit sur l'environnement et la santé humaine et permettant de lutter efficacement contre les insectes et/ou les arachnides, en particulier en réduisant le risque de développement de résistance.

Le travail des inventeurs a permis de mettre en évidence que certains monoesters d'acide gras pouvaient avantageusement être utilisés comme composé insecticide et/ou arachnicide.

Les monoesters d'acide gras peuvent parfois être utilisés dans des compositions pharmaceutiques ou phytopharmaceutiques comme adjuvant. A titre d'exemple, la demande EP 0839448 concerne une composition pesticide comportant un ingrédient actif pesticide et 15 à 40% en poids d'un monoester d'acide gras. Toutefois, leur utilisation en tant qu'insecticide et/ou arachnicide n'y est pas décrite.

La demande FR 2377154 concerne une combinaison synergique pédiculicide d'au moins un alcool aliphatique ou aryl-aliphatique et d'au moins un ester carboxylique aliphatique en C₄ à C₃₂ environ. En revanche, cette demande ne décrit pas l'utilisation en tant qu'insecticide des composés selon l'invention.

La présente invention concerne donc l'utilisation d'un certain type d'esters d'acide gras comme composés insecticides. Elle vise plus particulièrement l'utilisation d'un composé de formule (I) ou d'un mélange de composés de formule (I) : 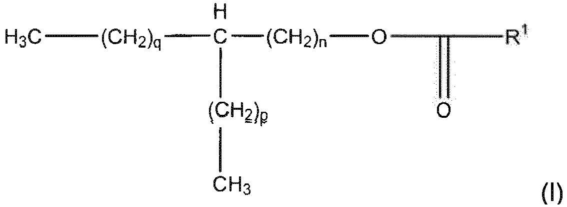 dans laquelle :
- R¹ est une chaîne alkyle comportant 11 atomes de carbone, q = p = 0 et n = 2, ou
- R¹ est une chaîne alkyle comportant 9 atomes de carbone, p = n = 1 et q = 3,
comme insecticide pour traiter les insectes de la famille des hémiptères, des lépidoptères, des diptères et/ou des coléoptères. Dans le cadre de la présente invention, les chaînes R¹ sont linéaires saturées non substituées. On notera que dans le cadre de la présente demande, et sauf stipulation contraire, les gammes de valeurs indiquées s'entendent bornes incluses.

Par insecticide, on entend une substance destinée à lutter, de manière préventive et/ou curative, contre les insectes.

Par lutter contre les insectes, on entend le fait de tuer les insectes adultes, leurs larves et/ou leurs œufs, de les repousser et/ou de contrôler leur prolifération. En particulier, le composé de formule (I) ou le mélange de composés de formule (I) peut être utilisé non seulement comme insecticides à usage agricole et/ou pour des espaces verts tels que des jardins, des parcs, mais également comme insecticides à usage humain ou vétérinaire, en particulier comme répulsifs (tels que les « anti-moustiques »).

Par arachnicide, on entend une substance destinée à lutter, de manière préventive et/ou curative, contre les arachnides, et plus particulièrement contre les araignées et les acariens. Avantageusement, l'arachnicide est un acaricide.

Par lutter contre les arachnides, on entend le fait de tuer les arachnides adultes, leurs larves et/ou leurs œufs, de les repousser et/ou de contrôler leur prolifération. En particulier, le composé de formule (I) ou le mélange de composés de formule (I) peut être utilisé non seulement comme arachnicide à usage agricole et/ou pour des espaces verts tels que des jardins, des parcs, mais également comme arachnicide à usage humain ou vétérinaire.

Par chaîne alkyle, on vise plus particulièrement une chaîne alkyle linéaire saturée non substituée.

Les composés de formule (I) et les mélanges de composés de formule (I) ont la propriété d'être facilement biodégradable et présente une faible toxicité tant à l'égard des humains que des animaux, autres que les insectes et arachnides.

Selon un premier mode de réalisation, on utilise comme insecticide un composé de formule (I) ou un mélange de composés de formule (I) dans laquelle R¹ est une chaîne alkyle comportant 11 atomes de carbone, q = p = 0 et n = 2.

Le composé de formule (I) est alors le dodécanoate de 3-méthylbutyle (également désigné sous les terminologies laurate d'isoamyle ou laurate d'isopentyle).

Ce composé, qui peut être issu de ressources renouvelables, est totalement et facilement biodégradable, non bio-accumulatif et non toxique sur les animaux autres que les insectes et les arachnides. En particulier, il est non toxique pour les organismes aquatiques et les microorganismes. Il est également non toxique pour les humains et ne présente pas non plus de risque d'irritation de la peau et des yeux. Le composé est donc plus sûr et sain pour l'agriculteur l'utilisant, pour la population vivant autour de l'exploitation traitée et pour l'environnement en général. En outre, le laurate d'isoamyle peut avantageusement être issus de ressources renouvelables. L'utilisation de ce composé en tant qu'insecticide aura donc un faible impact sur l'environnement.

Selon un second mode de réalisation, on utilise comme insecticide un composé de formule (I) ou un mélange de composés de formule (I) dans laquelle R¹ est une chaîne alkyle comportant 9 atomes de carbone, p = n = 1 et q = 3. Il s'agit alors du caprate de 2-éthylhexyle.

Comme indiqué ci-avant, l'insecticide selon l'invention présente un faible impact sur l'environnement. Avantageusement, le composé ou le mélange de composés de formule (I) est issu de ressources renouvelables et résulte par exemple de l'estérification de l'huile de palmiste et/ou de l'huile de coco par un alcool de formule (II) : 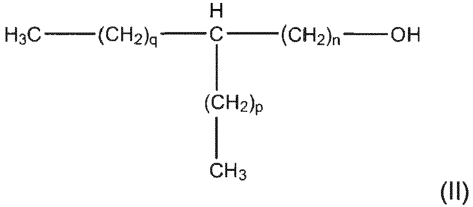 dans laquelle :
- q = p = 0 et n = 2, ou
- p = n = 1 et q = 3.

En général, l'huile de coco présente la composition en chaînes grasses saturées suivante: 5-9% C8, 5-10% C10; 44-53% C12; 13-19% C14; 8-11% C16; 1-3% C18, les pourcentages indiqués étant des pourcentages en poids sur le poids total de la composition et le chiffre suivant la lettre C indiquant le nombre de carbone dans la chaîne grasse saturée.

De même, en général, l'huile de palmiste présente la composition en chaînes grasses saturées suivante: 2-5% C8, 2-4% C10; 45-56% C12; 13-18% C14; 6-12% C16; 1-3% C18.

Selon un premier mode de réalisation, on utilise comme alcool un composé de formule (II) dans laquelle q = p = 0 et n = 2. L'alcool de formule (II) est le 3-méthylbutan-1-ol.

Selon un second mode de réalisation, on utilise comme alcool le 2-éthylhexan-1-ol.

De préférence, l'alcool de formule (II) est un mélange comportant environ 80% de 3-méthylbutan-1-ol et environ 20% de 2-méthylbutan-1-ol.

Le composé ou mélange insecticide selon l'invention peut être utilisé comme insecticides à usages agricoles et/ou pour des espaces verts tels que des jardins, des parcs, ou comme insecticides à usage humain ou vétérinaire.

Du fait de sa parfaite innocuité, le composé ou mélange de composés est tout à fait adapté pour être utilisé comme répulsif ou « anti-moustique » à usage humain ou vétérinaire.

Selon un mode de réalisation préférentiel, un tel répulsif se présente sous la forme d'un spray, dont la composition est destinée à être pulvérisée dans l'atmosphère (bombe aérosol), sur la peau ou les vêtements. Dans ce mode de réalisation, la composition comportant le composé ou mélange de composés est avantageusement sous la forme d'une émulsion.

Selon un autre mode de réalisation préférentiel, un tel répulsif se présente sous la forme d'un diffuseur, tel qu'un diffuseur à huiles essentielles, un diffuseur électrique ou plus simplement un bracelet diffuseur. Dans ce mode de réalisation, la composition comportant le composé ou mélange de composés est avantageusement sous la forme d'un concentré solide ou huileux.

De préférence, le composé ou mélange insecticide est utilisé comme insecticides à usages agricoles et/ou pour des espaces verts tels que des jardins, des parcs, pour traiter, à titre préventif ou curatif, les insectes de la famille des hémiptères, des lépidoptères, des diptères et/ou des coléoptères.

Parmi les hémiptères, on vise plus particulièrement le puceron des céréales (*Rhopalosiphum padi*), la punaise verte *(Nezara viridula)* et la mouche blanche (*Bemisia tabaci*).

Parmi les lépidoptères, on vise plus particulièrement la pyrale du maïs (*Ostrinia nubilalis*), la pyrale du haricot (*Etiella zinckenella*), les noctuelles (telles que *Spodoptera frugiperda* et *exigua*), le bombyx disparate (*Lymantria dispar*)*,* la teigne des crucifères (*Plutella xylostella*).

Parmi les diptères, on vise plus particulièrement la mouche grise des céréales (*Delia coarctata*)*.*

Parmi les coléoptères, on vise plus particulièrement la méligèthe (*Meligethes aeneus*), les altises (du chou, du lin, du maïs, etc.) et le doryphore (*Leptinotarsa decemlineata*).

Le composé ou mélange insecticide selon l'invention peut avantageusement être utilisé pour traiter, à titre préventif et/ou curatif, des cultures fruitières, ornementales, céréalières, potagères et/ou des oléagineux.

Par cultures fruitières, on entend plus particulièrement les arbustes et arbres fruitiers tels que les groseilliers, les fraisiers, les pêchers, les poiriers, les pommiers, le melon.

Par cultures ornementales, on entend plus particulièrement les arbres et arbustes d'ornement tels que les conifères, les arbres feuillus, les plantes ornementales et/ou florales, les arbustes fruitiers d'ornement et les plantes d'intérieurs. A titre d'exemple, on peut citer :
- pour les conifères: les pins, les épicéas, les mélèzes, les sapins,
- pour les arbres feuillus : les chênes, les charmes, les hêtres, les peupliers, les bouleaux, les saules, les érables, les tilleuls, les aulnes,
- les plantes ornementales et/ou florales : les cyclamens, les dahlias, les hortensias et les rosiers.

Par cultures céréalières, on entend plus particulièrement le maïs, le riz et les céréales à pailles telles que l'orge, l'avoine, le blé, le froment.

Par cultures potagères, on entend plus particulièrement les pommes de terre, les aubergines, les tomates, les poivrons, certaines crucifères dont les choux et les choux fleurs, les carottes, les courges, les concombres, les haricots verts, les betteraves et les légumineuses telles que pois, haricots, lentilles, féveroles.

Enfin, par culture des oléagineux, on entend plus particulièrement le colza (ou canola), le coton, le tournesol, le soja, le lin et le chanvre.

De manière plus spécifique, le composé ou mélange insecticide selon l'invention peut avantageusement être utilisé pour traiter, à titre préventif ou curatif :
- parmi les cultures fruitières :
   ∘ la pyrale du maïs infestant ou susceptible d'infester les cultures de melons ;
- parmi les cultures ornementales :
   ∘ la mouche blanche (*Bemisia tabaci*) infestant ou susceptibles d'infester les arbres et arbustes d'ornement, les plantes d'intérieur et les rosiers,
   ∘ -le bombyx disparate (*Lymantria dispar*) infestant ou susceptible d'infester les arbres feuillus tels que les chênes, les charmes, les hêtres, les peupliers, les bouleaux, les saules, les érables, les tilleuls, les aulnes et les conifères tels que les pins, les épicéas, les mélèzes et les sapins,
   ∘ la noctuelle de la betterave (*Spodoptera exigua*) infestant ou susceptible d'infester les plantes sous serres, en pot, telles que les chrysanthèmes, les gerberas, les rosiers ;
- parmi les cultures potagères :
   ∘ le doryphore (*Leptinotarsa decemlineata*) infestant ou susceptible d'infester des cultures telles que celles de pommes de terre, d'aubergines, de tomates,
   ∘ la mouche blanche (*Bemisia tabaci*) infestant ou susceptible d'infester des cultures telles que celles de tomates, de haricot verts,
   ∘ la teigne des crucifères (*Plutella xylostella*) infestant ou susceptible d'infester les cultures de choux et de choux fleurs,
   ∘ la punaise verte *(Nezara viridula)* infestant ou susceptible d'infester des cultures telles que celles d'aubergines, de concombres, de tomates, de poivrons et de haricots,
   ∘ la pyrale du haricot (*Etiella zinckenella*) infestant ou susceptible d'infester les cultures de légumineuses, telles que les pois, les haricots ou les lentilles,
   ∘ l'altise infestant ou susceptible d'infester des cultures telles que celles de choux,
   ∘ la noctuelle de la betterave (*Spodoptera exigua*) infestant ou susceptible d'infester les cultures de betteraves, de poivrons ;
- parmi les cultures céréalières :
   ∘ la mouche grise des céréales (*Delia coarctata*) infestant ou susceptible d'infester toute sorte de céréales,
   ∘ le puceron des céréales (*Rhopalosiphum padi*) infestant ou susceptible d'infester le riz et les céréales à pailles telles que l'orge, l'avoine, le blé, le froment,
   ∘ la pyrale du maïs (*Ostrinia nubilalis*) infestant ou susceptible d'infester le maïs, le tournesol, le chanvre,
   ∘ la noctuelle américaine du maïs (*Spodoptera frugiperda*) infestant ou susceptible d'infester le maïs, le riz, le sorgho,
   ∘ la punaise verte *(Nezara viridula)* infestant ou susceptible d'infester le riz,
   ∘ l'altise infestant ou susceptible d'infester le maïs ;
- parmi les cultures des oléagineux :
   ∘ la méligèthe (*Meligethes aeneus*) et la teigne des crucifères (*Plutella xylostella*) infestant ou susceptible d'infester le colza,
   ∘ la noctuelle américaine du maïs (*Spodoptera frugiperda*) et la mouche blanche (*Bemisia tabaci*) infestant ou susceptible d'infester le coton,
   ∘ la punaise verte *(Nezara viridula)* et la pyrale du haricot (*Etiella zinckenella*) infestant ou susceptible d'infester le soja,
   ∘ les altises infestant ou susceptibles d'infester le lin, le colza ;

De préférence, le composé ou mélange insecticide selon l'invention est utilisé pour traiter, à titre préventif ou curatif, le puceron des céréales, la méligèthe, la pyrale du maïs et/ou le doryphore.

La présente invention concerne également des compositions, en particulier des concentrés et des solutions, utilisables comme insecticides.

Un concentré selon l'invention comporte un composé insecticide ou un mélange de composés insecticides, le ou les composés étant choisi(s) parmi le groupe constitué par les composés de formule (I) : 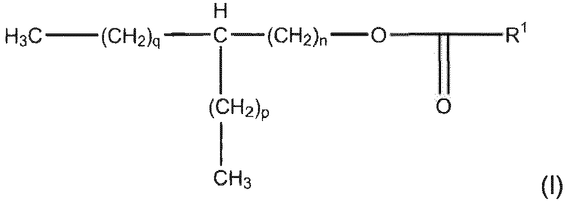 dans laquelle :
- R¹ est une chaîne alkyle comportant 11 atomes de carbone, q = p = 0 et n = 2, ou
- R¹ est une chaîne alkyle comportant 9 atomes de carbone, p = n = 1 et q = 3.

Par chaîne alkyle, on vise plus particulièrement une chaîne alkyle linéaire saturée non substituée.

Selon un premier mode de réalisation, on utilise comme insecticide, un composé de formule (I) ou un mélange de composés de formule (I) dans laquelle R¹ est une chaîne alkyle comportant 11 atomes de carbone, q = p = 0 et n = 2.

Le composé de formule (I) est alors le dodécanoate de 3-méthylbutyle.

Selon un second mode de réalisation, on utilise comme insecticide un composé de formule (I) ou un mélange de composés de formule (I) dans laquelle R¹ est une chaîne alkyle comportant 9 atomes de carbone, p = n = 1 et q = 3. Il s'agit alors du caprate de 2-éthylhexyle.

Par concentré, on vise plus particulièrement une composition ne comportant pas d'eau. Par « ne comportant pas d'eau », on entend un concentré dans lequel la présence éventuelle d'eau est uniquement due à la présence d'eau dans les éventuels composants du concentré (pas d'eau ajoutée).

Selon un premier mode de réalisation préférentiel, l'invention concerne un concentré comportant une quantité efficace d'un composé insecticide de formule (I) ou d'un mélange de composés insecticides de formule (I), un tensioactif et moins de 20% en poids d'un solvant aromatique hydrocarboné, le pourcentage en poids étant donné sur le poids total du concentré.

Par quantité efficace, on vise plus particulièrement une quantité strictement supérieure à 10% en poids sur le poids total du concentré, préférentiellement au moins 11%, plus préférentiellement, au moins 15%, plus préférentiellement encore au moins 30%.

Par solvant, on entend une substance dont la principale fonction est de dissoudre, de diluer ou d'extraire d'autres substances sans provoquer de modification chimique de ces substances et sans lui-même se modifier. Le concentré ne comportant pas d'eau, l'eau n'est pas considérée comme un solvant au sens de la présente invention.

Par solvant aromatique hydrocarboné, on entend plus particulièrement les alkylbenzènes (tels que le xylène et le tétraméthylbenzène), les alkylnaphtalènes (tels que le naphtalène, le méthylnaphtalène), le diphényl-éthane, le phénylxylyléthane et leurs mélanges.

Préférentiellement, la quantité de solvant aromatique hydrocarboné est de maximum 19% en poids sur le poids total du concentré, plus préférentiellement, maximum 15%, plus préférentiellement encore max 10%. Par exemple, la quantité de solvant aromatique hydrocarboné peut être de 0%.

Le concentré selon l'invention peut contenir un ou plusieurs tensioactif(s), ionique(s) et/ou non ionique(s), acceptable(s) en agriculture ou pharmaceutiquement acceptable. Des exemples de tensioactifs particulièrement avantageux sont les tensioactifs biosourcés et/ou les biosurfactants. Les biosurfactants sont des tensioactifs synthétisés par des microorganismes. Préférentiellement, le biosurfactant est un glycolipide, un glycoside ou leurs dérivés.

Le tensioactif peut être incorporé directement dans la composition ou via l'ajout d'un adjuvant.

Par « incorporé via l'ajout d'un adjuvant », cela signifie que l'adjuvant incorporé dans la composition consiste en ou comporte le tensioactif.

Par adjuvant, on entend un produit ou une préparation dépourvue d'activité pharmaceutique ou phytopharmaceutique, qui, associé(e) à un composé de formule (I) ou à un mélange de composés de formule (I) en améliore les qualités physiques, chimiques ou biologiques, en particulier leur activité insecticide, tout en limitant les effets néfastes et les impacts sur les êtres humains, la faune et la flore.

L'augmentation de l'activité insecticide des insecticides selon l'invention, peut par exemple résulter d'une meilleure vectorisation ou d'un meilleur ciblage de la partie à traiter et/ou d'une amélioration de la stabilité des insecticides, par exemple, en les protégeant des ultra-violets.

A titre d'exemple, on peut citer comme adjuvant consistant en ou comportant un ou plusieurs tensioactif(s) : un adjuvant à base d'esters méthyliques d'huiles végétales ou de graisses animales et en particulier les esters méthyliques d'huile de colza avec tensioactif(s) tels que l'Actirob B®, le Radiamix® ou le Vegestar® commercialisés par Novance®, un adjuvant à base de lécithine de soja et d'acide propionique tel que le Li-700® commercialisé par Agridyne®, un adjuvant à base de polymère d'amine grasse éthoxylée et de polysorbate 20 tel que le Surf 2000® commercialisé par Jouffray-Drillaud JD®, le polysorbate 20 tel que Tween® 20 commercialisé par Croda®, un adjuvant à base d'éther d'octylphénol octaglycol tel que l'Agral® Maxx ou l'Extravon® de Syngenta®, un adjuvant à base de nonylphénol polyéthoxylé tel que l'Agral® 90 de Syngenta®, un adjuvant à base d'esters de polyéthylène glycol d'alkylphénol tel que l'Emulsol® de Phyteurop®, un adjuvant à base d'alkylphénol (poly)oxyéthylène tel que l'Etaldyne Jardin de Scotts France ou le Rosemox® de Bayer® Cropscience France, un adjuvant à base de polyéther trisiloxane tel que le Break Thru® S 240 d'Evonik®, un adjuvant à base d'un ester de phosphate d'alcools gras polyoxyalkylés, des esters méthyliques d'acides gras et de l'acide oléique tel que le Dash® HC de BASF® Agro, un adjuvant à base de triglycérides éthoxylés tel que Cantor® de Vivagro et un adjuvant à base d'esters éthyliques d'huile de tournesol oléique et de tensioactifs tel que le TRS2® de SDP.

Selon un mode particulier de réalisation, l'adjuvant comporte un mélange d'esters d'acide gras, en particulier, des esters méthyliques d'huile de colza, et au moins un tensioactif, de préférence des tensioactifs ionique(s) et non ionique(s).

Avantageusement, le concentré est un concentré émulsifiable.

Ainsi formulé, le concentré selon l'invention présente l'avantage d'être un insecticide biodégradable, moins toxique pour l'environnement, pouvant même éventuellement être utilisé en agriculture biologique.

Selon un deuxième mode de réalisation préférentiel, le concentré comporte une quantité efficace d'un composé de formule (I) ou d'un mélange de composés de formule (I) et un tensioactif à l'exclusion des sels d'acide alkylarylsulfonique et d'acide alkylbiphénylsufonique.

De préférence, le tensioactif est un tensioactif biosourcé tel qu'un alkyl polyglycoside (APG) ou un alkyl polypentoside (APP), ou un biosurfactant tel qu'un glycolipide, un glycoside ou leurs dérivés.

Par quantité efficace, on vise plus particulièrement une quantité strictement supérieure à 10% en poids sur le poids total du concentré, préférentiellement au moins 11%, plus préférentiellement, au moins 15%, plus préférentiellement encore au moins 30%.

Les concentrés selon l'invention peuvent également comporter un solvant. Le solvant peut être minéral ou végétal, solide (par exemple, pour l'obtention de tablettes répulsives ou de compositions auto émulsifiables) ou liquide, acceptable en agriculture ou pharmaceutiquement acceptable.

Comme le tensioactif, le solvant peut être incorporé dans la composition seul ou via l'ajout d'un adjuvant.

A titre d'exemple, on peut citer comme adjuvant comportant un solvant, seul ou en combinaison avec d'autres composés : un adjuvant à base d'esters méthyliques d'huiles végétales ou de graisses animales tels que l'Actirob B®, le Radiamix® ou le Vegestar® (esters méthyliques d'huile de colza avec des tensioactif(s)) commercialisés par Novance® ou tel que le Radia® 7961 (esters méthyliques d'huile de colza), le Radia® 7064 (esters méthyliques d'huile de soja) ou le Radia® (esters éthyliques d'huile de tournesol oléique) commercialisé par Oleon®, un adjuvant à base d'un ester de phosphate d'alcools gras polyoxyalkylés, des esters méthyliques d'acides gras et de l'acide oléique tel que le Dash® HC de BASF® Agro, un adjuvant à base d'éthoxylate d'isodécyl alcool tel que Trend® 90 commercialisé par DuPont®, un adjuvant à base d'alcools terpéniques tel que Heliosol® commercialisé par Action Pin® et un adjuvant à base d'esters éthyliques d'huile de tournesol et de tensioactifs tel que le TRS2® de SDP.

De préférence, les concentrés selon l'invention comportent un adjuvant, acceptable en agriculture ou pharmaceutiquement acceptable.

Avantageusement, lorsque l'adjuvant est un adjuvant acceptable en agriculture, il peut comporter, outre un tensioactif et/ou un solvant éventuel, une protéine et/ou un ou plusieurs agents tels qu'un antimousse, un antigel, un épaississant, un agent mouillant, un rétenteur, un anti-rebond, un agent anti-dérive ou un stabilisant.

La plupart de ces adjuvants sont particulièrement utiles lorsque le concentré est destiné à être émulsionné dans de l'eau puis pulvérisé.

Par agent mouillant, on vise un adjuvant qui abaisse la tension superficielle de l'eau permettant ainsi l'étalement de la gouttelette sur la cible.

Par rétenteur, on vise un adjuvant favorisant le maintien des gouttelettes pulvérisées sur la cible au moment de l'impact.

Par anti-rebond, on vise un adjuvant permettant une meilleure accroche des gouttelettes pulvérisées sur la zone à traiter au moment de l'impact.

Par anti-dérive, on vise un adjuvant permettant de ne pas favoriser la création de gouttelettes trop fines (<100 - 150 µ) de façon à obtenir une meilleure précision dans le ciblage du produit pulvérisé en réduisant au maximum les pertes dans l'environnement ou vers les cultures voisines.

Selon un mode de réalisation particulier, le concentré comporte un composé insecticide de formule (I) ou un mélange de composés insecticides de formule (I), un mélange d'esters méthyliques d'huile de colza, et au moins un tensioactif. Un tel concentré est utilisable en agriculture.

Une quantité efficace de composé insecticide de formule (I) ou du mélange de composés insecticides de formule (I) est introduit dans le concentré, telle qu'une quantité strictement supérieure à 10% en poids sur le poids total du concentré, préférentiellement au moins 11%, plus préférentiellement, au moins 15%, plus préférentiellement encore au moins 30%.

Avantageusement, le concentré comporte au moins deux tensioactifs, ionique et non ionique. Les tensioactifs et un mélange d'esters méthyliques d'huile de colza sont préférentiellement introduits dans le concentré via l'ajout d'un adjuvant. A titre d'exemple, un tel adjuvant comporte avantageusement de 50 à 99% en volume d'esters méthylique d'huile de colza et 1 à 50% en volume d'un mélange de tensioactifs ionique et non ionique, les pourcentages en volume étant donné sur le volume total de l'adjuvant. L'adjuvant peut être choisi parmi l'Actirob B®, Radiamix® ou Vegestar®.

Avantageusement, les concentrés selon l'invention comportent un second composé insecticide et/ou arachnicide, avantageusement, à une teneur allant de 1 à 100% en poids du second composé insecticide et/ou arachnicide sur le poids du premier composé insecticide et/ou arachnicide, de préférence de 10 à 50% en poids.

Le second composé insecticide et/ou arachnicide peut être choisi parmi le groupe constitué par : les pyréthrines et leurs dérivés, les pyréthrinoïdes, les composés organophosphorés, les carbamates, les néonicotinoïdes, les anthranilamides, les benzoylurées, les oxadiazines, les organochlorés, les phénylpyrazoles, les arylpyrroles, les avermectines, les spynosynes, les régulateurs de croissance et les mimétiques d'hormones juvéniles.

L'ajout d'un second insecticide et/ou arachnicide dans les concentrés selon l'invention permet l'obtention d'une composition insecticide et/ou arachnicide dont le pouvoir insecticide et/ou arachnicide à l'égard d'un insecte et/ou arachnide particulier est renforcé ou dont le spectre des insectes et/ou arachnides visés est élargi. Alternativement, il est également possible de réduire la teneur en insecticide et/ou arachnicide traditionnel (potentiellement plus toxique) ou non biologique, permettant ainsi l'obtention d'un insecticide et/ou arachnicide plus sain ou moins toxique pour l'agriculteur, le voisinage des champs traités et l'environnement. Une combinaison d'actifs insecticides et/ou arachnicides permet également de réduire les résistances des insectes et/ou arachnides aux insecticides et/ou arachnicides, grâce par exemple, à la combinaison de deux insecticides et/ou arachnicides présentant des modes d'action différents. L'homme du métier est familier avec la formulation de tels insecticides et/ou arachnicides.

Par pyréthrines et leur dérivés, on vise plus particulièrement les Pyréthrines I et II (utilisable en agriculture biologique), les Jasmolines I et II et les Cinérines I et II.

Par pyréthrinoïdes, on vise plus particulièrement les actifs insecticides suivant : Alléthrine, d-Alléthrine, Bioalléthrine, Alpha-méthrine, Bifenthrine, Bioresméthrine, Cycloprothrine, Cyfluthrine, Beta-cyfluthrine, Cyhalothrine, Gamma-cyhalothrine, Lambda-cyhalothrine (bio), d,d-trans-Cyphénothrine, Cyperméthrine, Alpha-cyperméthrine, Beta-cyperméthrine, Zeta-cyperméthrine, Cyphénothrine, Deltaméthrine (bio), Dépalléthrine, Diméfluthrine, Empenthrine, Esbiothrine, Esdépalléthrine, Esfenvalérate, Etofenprox, Fenpropathrine, Fenvalérate, Flucythrinate, Fluvalinate, Tau-fluvalinate, Imiprothrine, Kadéthrine, Métofluthrine, Perméthrine, Phénothrine, d-Phénothrine (Sumithrine), Pralléthrine, Profluthrine, Resméthrine, Silafluofen, Téfluthrine, Tétraméthrine, d-Tétraméthrine, Tralométhrine et Transfluthrine.

Par composés organophosphorés, on vise plus particulièrement les actifs insecticides suivant : Acéphate, Azaméthiphos, Azinphos-méthyle, Bromophos-éthyle, Chlorfenvinphos, Chlorpyrifos, Chlorpyrifos-méthyle, Coumafos, Cyanophos, Déméton, Diazinon, Dichlofenthion, Dichlorvos, Dicrotophos, Diméthoate, disulfoton, Ethion, Ethoprophos, Etrimfos, Fenchlorphos, Fénitrothion, Fenthion, Fonofos, Formothion, Heptenophos, lodofenphos, Isofenphos, Isoxathion, Malathion, Mecarbam, Méthamidophos, Méthidation, Mévinphos, Monocrotophos, Naled, Ométhoate, Oxydéméton-méthyle, Paraoxon, Parathion, Parathion-éthyle, Parathion-méthyle, Phenthoate, Phosalone, Phosmet, Phosphamidon, Phorate Phoxim, Pirimiphos-méthyle, Pirimiphos-éthyle, Profénofos, Propétamphos, Protiophos, Pyraclofos, Pyridapenthion, Quinalphos, Sulfotep, Sulprophos, Tebupimimfos, Téméphos, Terbufos, Tetrachlorvinphos, Thiométon, Triazophos, Trichlorfon et Vamidothion.

Par carbamates, on vise plus particulièrement les actifs insecticides suivant: Alanycarbe, Aldicarbe, Aminocarbe Bendiocarbe, Benfuracarbe, Carbaryle, Carbofuran, Carbosulfan, Diallate, Dimétan, Dimétilan, Dioxacarbe, Ethiophencarbe, Fenobucarbe (BPMC), Fénoxycarbe, Formétanate, Formétanate hydrochloride, Furathiocarbe, Isocarbe, Isoprocarbe (MICP), Mercaptodiméthur, Méthiocarbe, Méthomyle, Métolcarbe (MTMC), Mexacarbe, Oxamyle, Pirimicarbe, Promecarbe, Propoxur, Thiodicarbe, Thiofanoxe et Triazamate.

Par néonicotinoïdes, on vise plus particulièrement les actifs insecticides Acétamipride, Clothianidine, Dinotéfuran, Imidaclopride, Nitenpyram, Sulfoxaflor, Thiaclopride et Thiaméthoxam.

Par anthranilamides, on vise plus particulièrement les actifs insecticides Chlorantraniliprole et Cyantraniliprole.

Par benzoyl urées, on vise plus particulièrement les actifs insecticides Chlorfluazuron, Diflubenzuron, Flucycloxuron, Flufénoxuron, Hexaflumuron, Lufénuron, Novaluron, Téflubenzuron et Triflumuron.

Par oxadiazines, on vise plus particulièrement l'Indoxacarbe.

Par organochlorés, on vise plus particulièrement les actifs insecticides Chlordane, DDT, Endosulfan, Lindane et Méthoxychlor.

Par phénylpyrazoles, on vise plus particulièrement les actifs insecticides Ethiprole et Fipronil.

Par arylpyrroles, on vise plus particulièrement le Chlorfénapyr.

Par avermectines, on vise plus particulièrement les actifs insecticides Abamectine, Aversectin C, Doramectin, Emamectin, Eprinomectin, Ivermectin, Selamectin, de préférence Abamectin, Aversectin C et Emamectin, plus préférentiellement et Abamectin.

Par spinosynes, on vise plus particulièrement les actifs insecticides Spinetoram, et Spinosad.

Par régulateurs de croissance, on vise plus particulièrement les actifs insecticides Azadirachtine, Cyromazine et Dicyclanil.

Par mimétiques d'hormones juvéniles, on vise plus particulièrement les actifs insecticides Hydroprène, Méthoprème, S-méthoprène et Pyriproxyfène.

Les composés et actifs insecticides et/ou arachnicides mentionnés ci-avant peuvent également se présenter sous forme de sel(s) (tel que par exemple, le benzoate d'Emamectin) ou sous forme de mélange desdits composés ou de leur(s) sel(s).

Les concentrés selon l'invention peuvent également comporter un biopesticide, c'est-à-dire une substance pesticide d'origine naturelle, une substance pesticide produite par des plantes contenant du matériel génétique ajouté (« plant incorporated protectants ») ou un microorganisme pesticide.

A titre d'exemple, on peut citer :
- des bactéries telles que *Bacillus thuringiensis* et/ou la toxine *Bt, Bacillus subtilis,*
- les champignons entomopathogènes tels que *Beauveria bassiana, Lecanicillium spp., Metarhizium spp.,*
- les phéromones d'insectes, ainsi que toute autre substance émise par un insecte,
- les produits issus de la fermentation, tel que par exemple l'actif insecticide Spinosad indiqué ci-avant,
- le chitosan,
- des minéraux d'origine naturelle tels que le bicarbonate de sodium, les terres de diatomées, le silicate de potassium,
- et plus généralement, tout produit naturel dérivé de plante et/ou d'extraits de plantes tel que des alkaloïdes, terpénoïdes, dérivés phénoliques, huiles végétales (huile de colza, huile de neem), etc.

Les concentrés peuvent comporter optionnellement un produit fertilisant pour plantes tel qu'un engrais (microorganismes, substances organiques ou minérales destinées à apporter aux plantes des éléments nutritifs additionnels, en particulier de l'azote, du phosphore, du potassium, du soufre, du magnésium et/ou du calcium), ou un amendement (composé ou matériau destiné à améliorer la qualité des sols).

Ils peuvent également comporter optionnellement un autre composé biologiquement actif tel qu'un nématicide et/ou un bactéricide.

Le concentré se présente avantageusement sous forme liquide, sous forme de gel ou sous forme solide telle qu'une poudre, des granulés, un film, ces formulations étant préférentiellement émulsifiables ou dispersibles dans de l'eau.

Selon un mode de réalisation particulier, le concentré consiste en :
- un composé insecticide de formule (I) ou un mélange de composés insecticides de formule (I),
- un ou plusieurs tensioactif(s),
- optionnellement un ou plusieurs solvant(s), avec la condition que si le concentré comporte un ou plusieurs solvant(s) aromatique(s) hydrocarboné(e)(s), la teneur en solvant(s) aromatique(s) hydrocarboné(e)(s) est inférieure à 20% en poids,
- optionnellement un ou plusieurs adjuvant(s),
- optionnellement un ou plusieurs autre(s) composé(s) insecticide(s) et/ou arachnicide(s), de préférence un second composé insecticide et/ou arachnicide.

Selon un autre mode de réalisation particulier, le concentré consiste en :
- un composé insecticide de formule (I) ou un mélange de composés insecticides de formule (I),
- un ou plusieurs tensioactif(s), à l'exclusion des sels d'acide alkylarylsulfonique et d'acide alkylbiphenylsufonique,
- optionnellement un ou plusieurs solvant(s),
- optionnellement un ou plusieurs adjuvant(s),
- optionnellement un ou plusieurs autre(s) composé(s) insecticide(s) et/ou arachnicide(s), de préférence un second composé insecticide et/ou arachnicide.

Les définitions, teneurs, tensioactif(s), solvant(s), adjuvant et second composé insecticide préférentielles mentionnées ci-avant sont également applicables à ces deux modes de réalisation particuliers.

On notera que les concentrés selon l'invention se distinguent des concentrés utilisables dans le domaine de la cosmétique en ce qu'il ne comporte pas d'émollient, tels que des mono, di ou tri-glycérides.

Selon un mode de réalisation particulier, dans les concentrés selon l'invention,

le composé insecticide de formule (I) est le dodécanoate de 3-méthylbutyle (laurate d'isoamyle). Des essais ont permis de démontrer qu'un concentré comportant du laurate d'isoamyle avait une activité insecticide similaire à un insecticide commercial comportant de la Lamba-cyhalothrine, tout en étant moins toxique, en particulier, du point de vue de la toxicité aquatique et de la toxicité chez l'humain (par inhalation, irritant pour la peau et les yeux).

L'invention est également relative à une solution insecticide comportant un concentré selon l'invention et de l'eau et à un procédé de préparation de la solution insecticide selon l'invention, par émulsion, dispersion ou dilution d'un concentré selon l'invention.

Avantageusement, le ratio en volume du concentré selon l'invention sur l'eau est compris entre 1 :400 et 1 :20, de préférence, entre 1 :300 et 1 :30, plus préférentiellement encore entre 1 :250 et 1 :40.

La solution, prête à l'emploi, se présente avantageusement sous la forme d'une émulsion, d'une dispersion ou d'une suspension.

Préférentiellement, la solution est sous la forme d'une émulsion pour pulvérisation. Une telle formulation permet une répartition des actifs insecticides de manière homogène sur la zone à traiter lors de l'application.

Optionnellement, il est possible lors de la préparation de la solution d'ajouter un adjuvant extemporané.

Les concentrés et solution selon l'invention sont utilisables comme insecticides, de manière préventive ou curative.

L'invention concerne enfin deux procédés de lutte contre les insectes de la famille des hémiptères, des lépidoptères, des diptères et/ou des coléoptères.

Selon un premier procédé, on met en contact avec une plante ou une partie de celle-ci, devant être traitée, une dose efficace d'un composé de formule (I), d'un mélange de composés de formule (I), d'un concentré selon l'invention, ou d'une solution selon l'invention.

Par « plante », on désigne tout membre du règne « Plantae », quel que soit son stade de développement tel que la graine, le bulbe, la plantule ou la plante adulte. Par partie de plante, on vise plus particulièrement les feuilles, les tiges, les fleurs, les fruits, les épis, les bourgeons, les racines, les tubercules, etc.

Selon un second procédé de lutte contre les insectes, on met en contact le sol devant être traité avec une dose efficace d'un composé de formule (I), d'un mélange de composés de formule (I), d'un concentré selon l'invention, ou d'une solution selon l'invention.

Les traitements peuvent être effectués à titre préventif et/ou curatif, de préférence par pulvérisation.

En général, une dose efficace allant de 0,2 à 10L/ha d'un composé de formule (I) ou un mélange de composés de formule (I) est appliquée.

En particulier, les procédés selon l'invention sont plus particulièrement adaptés pour traiter des plantes et des sols destinés à recevoir des plantes dans des cultures fruitières, ornementales, céréalières, potagères et/ou des oléagineux telles que décrite ci-avant.

Les insectes à traiter sont de la famille des hémiptères, des lépidoptères, des diptères et/ou des coléoptères, tels que décrit ci-avant.

L'invention sera mieux comprise au vu des exemples qui suivent donnés à titre illustratif, avec référence aux figures, qui représentent respectivement :
- Sur la figure 1, est représenté un diagramme illustrant les résultats d'efficacité insecticide d'une composition comportant uniquement un adjuvant en comparaison avec ceux d'une composition comportant le même adjuvant avec un produit insecticide selon l'invention,
- Sur la figure 2, est représenté un diagramme illustrant l'effet dose-réponse de l'efficacité insecticide d'une composition comportant un produit insecticide selon l'invention à différentes doses.

### Exemple 1 : Compositions et leur préparation

1.a) Une composition selon l'invention est obtenue en mélangeant 0 ,1 mL d'un adjuvant Actirob B® (adjuvant comportant des esters méthyliques d'huile de colza avec des tensioactifs ioniques et non ioniques), à 0,9 mL de dodécanoate de 3-méthylbutyle (également désigné sous l'appellation de laurate d'isoamyle). Ce mélange est agité vigoureusement pendant 30 secondes à la main puis 30 secondes par un agitateur Vortex. Le mélange est ensuite émulsifié dans l'eau juste avant son utilisation.
1.b) Une composition selon l'invention est obtenue en mélangeant 0,1 mL d'un adjuvant Actirob B® à 0,9 mL de caprate de 2-éthylhéxyle selon le procédé décrit en 1.a).
1.c) A titre de comparaison, une composition est obtenue en mélangeant 0,1 mL d'un adjuvant Actirob B® à 0,9 mL de palmitate de 2-éthylhexyle selon le procédé décrit en 1.a).

### Exemple 2 : Efficacité sur les pucerons (Rhopalosiphum padi)

Le test a été mené sur du blé infecté par des pucerons.

### Préparation de la population de pucerons

Les pucerons *Rhopalosiphum padi* sont élevés en laboratoire sur du blé. Les conditions d'élevage sont les suivantes : 14h à la lumière à 18 °C, et 10 h dans le noir à 15 °C sous une humidité relative (RH) de 60-70 %.

### Préparation des plantules

Le blé « *Triticum aestivum* » de la variété Apache est cultivé dans les conditions suivantes : 14h à la lumière à 18 °C, et 10 h dans le noir à 15 °C sous une humidité relative (RH) de 60-70 %. Pour les expériences, des plantules ayant atteint le stade 12 sur l'échelle BBCH, soit 2 feuilles, sont choisies.

### Expérience 2.a)

A J-1, 3 pucerons adultes ont été déposés sur chaque plantule de blé (environ 30 plantules par dose testée). Les plantules sont maintenues 14 h à la lumière à 18 °C, et 10 h dans le noir à 15 °C sous une humidité relative (RH) de 60-70 %.

Les plantules sont traitées le jour J avec une composition telle que décrite dans l'exemple 1.a). Des émulsions comportant les doses suivantes de laurate d'isoamyle ont été testées : 1,35 L/ha (N/4), 2,7 L/ha (N/2), 5,7 L/ha (N), 10,8 L/ha (2N) et 16,2 L/ha (3N). Deux contrôles ont été effectués : un premier contrôle avec de l'eau et un second contrôle avec l'adjuvant, testé seul, à une dose de 1,8 L/ha ce qui correspond à la dose d'adjuvant contenue dans la dose 3N de laurate d'isoamyle. Le volume de composition pulvérisée correspond à l'équivalent de 200L/ha.

Pour le traitement, les buses sont utilisées pour pulvériser les différentes émulsions et avoir une bonne répartition des gouttes sur les plantes (buse à jet plat TeeJet 110015VS utilisée sur un pulvérisateur de laboratoire Euro-Pulvé).

### Résultats 2.a)

A J+1, J+3, J+7, les adultes et larves présents sur chaque plantule sont comptés.

L'analyse statistique est réalisée avec XLSTATS et une analyse de variance avec le test de Newman-Keuls et de Dunnett. Les tests de Dunnett utilisés en post-hoc à une ANOVA, donne une probabilité de 0,00.

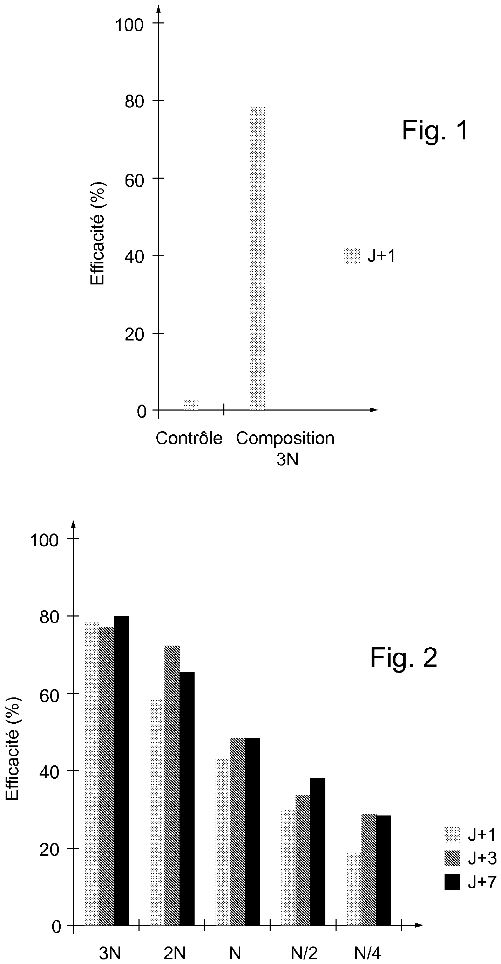

Les résultats sont présentés en figures 1 et 2. Sur la figure 1, on peut constater l'effet insecticide très limité de l'adjuvant sur le traitement de la plantule. Par ailleurs, sur la figure 2, on peut constater que le laurate d'isoamyle présente une efficacité de presque 80% à la dose 3N.

### Expérience 2.b)

A J-1, 3 pucerons adultes ont été déposés sur chaque plantule de blé (environ 30 plantules par dose testée). Les plantules sont maintenues 14 h à la lumière à 18 °C, et 10 h dans le noir à 15 °C sous une humidité relative (RH) de 60-70 %.

Les plantules sont traitées le jour J avec une composition telle que décrite dans l'exemple 1.b). Une émulsion comportant la dose suivante de caprate de 2-éthylhexyle a été testée : 16,2 L/ha (3N). Deux contrôles ont été effectués : un premier contrôle avec de l'eau et un second contrôle avec l'adjuvant, testé seul, à une dose de 1,8 L/ha ce qui correspond à la dose d'adjuvant contenue dans la dose 3N de caprate de 2-éthylhexyle. Le volume de composition pulvérisée correspond à l'équivalent de 200L/ha.

Pour le traitement, les buses sont utilisées pour pulvériser les différentes émulsions et avoir une bonne répartition des gouttes sur les plantes (buse à jet plat TeeJet 110015VS utilisée sur un pulvérisateur de laboratoire Euro-Pulvé).

### Résultats 2.b)

A J+1, J+3, J+7, les adultes et larves présents sur chaque plantule sont comptés.

L'analyse statistique est réalisée avec XLSTATS et une analyse de variance avec le test de Newman-Keuls et de Dunnett. Les tests de Dunnett utilisés en post-hoc à une ANOVA, donne une probabilité de 0,00.

L'effet insecticide de l'adjuvant est cette fois encore très limité sur le traitement de la plantule. Par ailleurs, on peut constater que le caprate de 2-éthylhexyle présente une efficacité de presque 55% à la dose 3N à J+1.

### Expérience 2.c)

A J-1, 3 pucerons adultes ont été déposés sur chaque plantule de blé (environ 30 plantules par dose testée). Les plantules sont maintenues 14 h à la lumière à 18 °C, et 10 h dans le noir à 15 °C sous une humidité relative (RH) de 60-70 %.

Les plantules sont traitées le jour J avec une composition telle que décrite dans l'exemple 1.c). Une émulsion comportant la dose suivante de palmitate de 2-éthylhexyle a été testée : 16,2 L/ha (3N). Deux contrôles ont été effectués : un premier contrôle avec de l'eau et un second contrôle avec l'adjuvant, testé seul, à une dose de 1,8 L/ha ce qui correspond à la dose d'adjuvant contenue dans la dose 3N de palmitate de 2-éthylhexyle. Le volume de composition pulvérisée correspond à l'équivalent de 200L/ha.

Pour le traitement, les buses sont utilisées pour pulvériser les différentes émulsions et avoir une bonne répartition des gouttes sur les plantes (buse à jet plat TeeJet 110015VS utilisée sur un pulvérisateur de laboratoire Euro-Pulvé).

### Résultats 2.c)

A J+1, J+3, J+7, les adultes et larves présents sur chaque plantule sont comptés.

L'analyse statistique est réalisée avec XLSTATS et une analyse de variance avec le test de Newman-Keuls et de Dunnett. Les tests de Dunnett utilisés en post-hoc à une ANOVA, donne une probabilité de 0,00.

L'effet insecticide de l'adjuvant est cette fois encore très limité sur le traitement de la plantule. Par ailleurs, on peut constater que le palmitate de 2-éthylhexyle présente une efficacité d'environ 10% à la dose 3N à J+1.

### Exemple 3 : Efficacité sur la pyrale du maïs (Ostrinia nubilalis)

Le test a été mené sur du maïs infecté par la pyrale du maïs.

### Préparation de la population de pyrales

Des ooplaques d'*Ostrinia nubilalis* sont maintenues dans des conditions contrôlées de laboratoire pour permettre le développement des œufs. Les œufs sont maintenus pendant 14h à la lumière et à 18°C et pendant 10h dans la nuit à 15°C avec une humidité relative oscillant de 60 à 70 %.

Après éclosion, les larves au stade 1° et 2° instar sont utilisées pour l'expérimentation.

### Préparation des plantules

Le maïs « *Zea Mays* » de la variété DK315 est cultivé dans les conditions suivantes : 14h à la lumière à 18 °C, et 10 h dans le noir à 15 °C sous une humidité relative (RH) de 60-70 %.

### Expérience

A J-1, 3 feuilles de maïs sont prélevées sur les plantules et introduites dans des boîtes en plastiques aérées. 10 larves sont alors déposées sur les feuilles de maïs dans chaque boîte, l'essai étant reproduit trois fois. Les larves sont maintenues 14 h à la lumière à 18 °C, et 10 h dans le noir à 15 °C sous une humidité relative (RH) de 60-70 %.

Les feuilles sont traitées le jour J avec une composition telle que décrite dans l'exemple 1.a) à une dose de laurate d'isoamyle équivalente à 6,0 L/ha.

Pour le traitement, les buses sont utilisées pour pulvériser la composition de l'exemple 1.a) et avoir une bonne répartition des gouttes sur les plantes (buse à jet plat TeeJet 110015VS utilisée sur un pulvérisateur de laboratoire Euro-Pulvé). Le volume de composition pulvérisée correspond à l'équivalent de 200L/ha.

### Résultats

A J+3, les larves et adultes présents dans chaque boîte sont comptés.

L'analyse statistique est réalisée avec XLSTATS et une analyse de variance avec le test de Newman-Keuls et de Dunnett. Les tests de Dunnett utilisés en post-hoc à une ANOVA, donne une probabilité de 0,05.

A une dose de 6,0 L/ha de laurate d'isoamyle, on obtient 19,5% d'efficacité sur les larves et adultes de la pyrale du maïs.

### Exemple 4 : Efficacité sur les méligèthes

L'efficacité du laurate d'isoamyle sur les méligèthes a été évaluée selon un protocole basé sur la méthode n°11 de l'IRAC (Insecticide Résistance Action Committee), version 3, juin 2009.

### Préparation de la population de méligèthes

Les méligèthes sont collectées sur différentes localisations sur des champs infestés. Elles sont ensuite stockées dans des boîtes plastiques aérées, dans lesquelles un papier sec a été placé au fond de la boîte. Quelques feuilles de colza ainsi que 2 à 3 colza en fleur sont ajoutées comme source de nourriture. Comme il est important que les méligèthes ne soient pas sujet à des températures excessives, à des conditions d'humidité ou autres source du stress après collecte, elles sont maintenues dans les conditions suivantes : 14h à la lumière à 18 °C, et 10 h dans le noir à 15 °C sous une humidité relative (RH) de 60-70 %.

### Préparation des solutions à tester

Le produit laurate d'isoamyle a été solubilisé dans de l'acétone. Chaque solution a ensuite été déposée de manière homogène sur l'intérieur d'une fiole en verre de 2 cm de diamètre et de 4 cm de hauteur, jusqu'à totale évaporation de l'acétone. La quantité d'insecticide apporté par 0,5 mL de solution à l'acétone est calculée de manière à ce qu'une unité de surface de la fiole soit couverte avec la même quantité d'insecticide que celle portée par la même unité de surface d'une feuille appartenant à un champ ayant subi un traitement à 200L/ha.

### Expérience

10 méligèthes sont introduites dans chaque fiole, chaque essai étant effectué deux fois.

La mortalité est évaluée après 24h.

### Résultat

Le tableau 1 ci-après montre l'efficacité obtenue selon la dose testée à 24h.

**Tableau 1**

| | | **Efficacité (%)** | |
|---|---|---|---|
| Produit | Dose L/ha | **Méligèthes** | |
| | | ***Meligethes aeneus*** | |
| | | Sensibles | Souches résistantes au pyréthrinoïdes |
| Laurate d'isoamyle | 2,0 L/ha | 55% | 40% |
| | 4,0 L/ha | 90% | 75% |

### Exemple 5 : Efficacité sur le doryphore (Leptinotarsa decemlineata)

L'efficacité du laurate d'isoamyle sur les doryphores a été évaluée selon un protocole basé sur la méthode n°7 de l'IRAC (Insecticide Résistance Action Committee), version 3, juin 2009.

### Préparation de la population de doryphores

Des œufs de doryphores sont récupérés en plein champ et déposés sur la face inférieure (abaxiale) de feuilles de pommes de terre. Après 4 à 15 jours, l'éclosion des œufs a lieu donnant naissant à des larves rouge-brun foncées.

### Préparation et traitement des feuilles de pommes de terre

Des feuilles de pommes de terre sont traitées par trempage pendant 5s dans avec une composition telle que décrite dans l'exemple 1.a) à une dose de laurate d'isoamyle équivalente à 6,0 L/ha.

### Expérience

Les feuilles de pommes de terre sont infestées, sur leur face abaxiale avec 5 larves de doryphore au stade L4. Chaque essai est répété une seconde fois.

### Résultats

La mortalité est évaluée après 5h. A une dose de 6,0 L/ha de laurate d'isoamyle, on obtient 10% d'efficacité sur les larves de doryphore.

## Revendications

1. Utilisation d'un composé de formule (I) ou d'un mélange de composés de formule (I) : 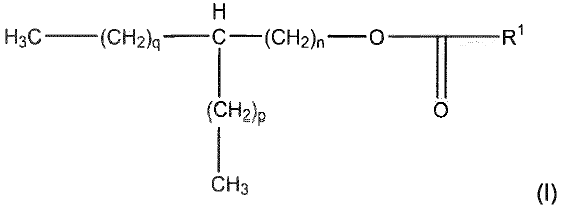 dans laquelle :
- R¹ est une chaîne alkyle linéaire saturée non substituée comportant 11 atomes de carbone, q = p = 0 et n = 2, ou
- R¹ est une chaîne alkyle linéaire saturée non substituée comportant 9 atomes de carbone, p = n = 1 et q = 3, comme insecticide pour traiter les insectes de la famille des hémiptères, des lépidoptères, des diptères et/ou des coléoptères.

2. Utilisation selon la revendication 1, pour traiter des cultures fruitières, ornementales, céréalières, potagères et/ou des oléagineux.

3. Concentré ne comportant pas d'eau comportant une quantité efficace d'un composé insecticide de formule (I) telle que définie dans la revendication 1 ou d'un mélange de composés insecticides de formule (I), un tensioactif et moins de 20% en poids d'un solvant aromatique hydrocarboné.

4. Concentré comportant une quantité efficace d'un composé insecticide de formule (I) ou d'un mélange de composés insecticides de formule (I) et un tensioactif à l'exclusion des sels d'acide alkylarylsulfonique et d'acide alkylbiphénylsufonique.

5. Concentré selon la revendication 4, dans lequel le tensioactif est un biosurfactant.

6. Concentré selon l'une quelconque des revendications 3 à 5, comportant un second composé insecticide.

7. Concentré selon l'une quelconque des revendications 3 à 6, dans laquelle le composé de formule (I) est le dodécanoate de 3-méthylbutyle.

8. Solution insecticide comportant un concentré selon l'une quelconque des revendications 3 à 7 et de l'eau.

9. Procédé de préparation d'une solution selon la revendication 8, par émulsion, dispersion ou dilution du concentré selon l'une quelconque des revendications 3 à 7 dans de l'eau.

10. Utilisation non thérapeutique d'un concentré selon l'une quelconque des revendications 3 à 7, ou de la solution selon la revendication 8, comme insecticide.

11. Procédé pour lutter contre les insectes de la famille des hémiptères, des lépidoptères, des diptères et/ou des coléoptères **caractérisé en ce qu'**on met en contact avec une plante ou une partie de celle-ci, devant être traitée, une dose efficace d'un composé de formule (I), d'un mélange de composés de formule (I), d'un concentré selon l'une quelconque des revendications 3 à 7, ou de la solution selon la revendication 8.

12. Procédé pour lutter contre les insectes de la famille des hémiptères, des lépidoptères, des diptères et/ou des coléoptères **caractérisé en ce qu'**on met en contact le sol devant être traité avec une dose efficace d'un composé de formule (I), d'un mélange de composés de formule (I), d'un concentré selon l'une quelconque des revendications 3 à 7, ou de la solution selon la revendication 8.

## Patentansprüche

1. Verwendung einer Verbindung der Formel (I) oder eines Gemischs von Verbindungen der Formel (I): 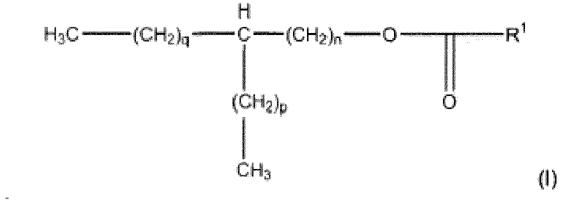 worin:
- R¹ eine lineare, gesättigte, nichtsubstituierte Alkylkette ist, die 11 Kohlenstoffatome umfasst, q = p = 0 und n = 2, oder
- R¹ eine lineare, gesättigte, nichtsubstituierte Alkylkette ist, die 9 Kohlenstoffatome umfasst, p = n = 1 und q = 3,
als Insektizid, um Insekten der Familie der Hemiptera, Lepidoptera, Diptera und/oder Coleoptera zu behandeln.

2. Verwendung nach Anspruch 1, um Obst-, Zierpflanzen-, Getreide-, Gemüse- und/oder Ölsaatanbau zu behandeln.

3. Nicht wasserhaltiges Konzentrat, das eine wirksame Menge einer Insektizidverbindung der Formel (I) wie in Anspruch 1 definiert oder eines Gemischs von Insektizidverbindungen der Formel (I), ein Tensid und weniger als 20 Gew.-% eines aromatischen Kohlenwasserstofflösungsmittels umfasst.

4. Konzentrat, das eine wirksame Menge einer Insektizidverbindung der Formel (I) oder eines Gemischs von Insektizidverbindungen der Formel (I) und ein Tensid unter Ausschluss von Alkylarylsulfonsäure- und Alkylbiphenylsulfonsäuresalzen umfasst.

5. Konzentrat nach Anspruch 4, worin das Tensid ein Biotensid ist.

6. Konzentrat nach einem der Ansprüche 3 bis 5, eine zweite Insektizidverbindung umfassend.

7. Konzentrat nach einem der Ansprüche 3 bis 6, worin die Verbindung der Formel (I) 3-Methylbutyl-Dodecanoat ist.

8. Insektizidlösung, umfassend ein Konzentrat nach einem der Ansprüche 3 bis 7 und Wasser.

9. Verfahren zum Aufbereiten einer Lösung nach Anspruch 8 durch Emulgieren, Dispergieren oder Verdünnen des Konzentrats nach einem der Ansprüche 3 bis 7 in Wasser.

10. Nichttherapeutische Verwendung eines Konzentrats nach einem der Ansprüche 3 bis 7 oder der Lösung nach Anspruch 8 als Insektizid.

11. Verfahren zum Bekämpfen von Insekten der Familie der Hemiptera, Lepidoptera, Diptera und/oder Coleoptera, **dadurch gekennzeichnet, dass** eine wirksame Dosis einer Verbindung der Formel (I), eines Gemischs von Verbindungen der Formel (I), eines Konzentrats nach einem der Ansprüche 3 bis 7 oder der Lösung nach Anspruch 8 mit einer Pflanze oder einem Teil von dieser, die bzw. das behandelt werden soll, in Kontakt gebracht wird.

12. Verfahren zum Bekämpfen von Insekten der Familie der Hemiptera, Lepidoptera, Diptera und/oder Coleoptera, **dadurch gekennzeichnet, dass** eine wirksame Dosis einer Verbindung der Formel (I), eines Gemischs von Verbindungen der Formel (I), eines Konzentrats nach einem der Ansprüche 3 bis 7 oder der Lösung nach Anspruch 8 mit dem Boden, der behandelt werden soll, in Kontakt gebracht wird.

## Claims

1. Use of a compound of formula (I) or of a mixture of compounds of formula (I) 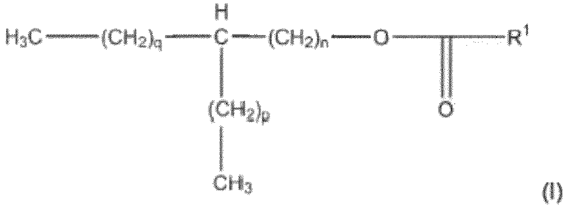 wherein:
- R¹ is an unsubstituted saturated linear alkyl chain including 11 carbon atoms, q = p = 0 and n = 2, or
- R¹ is an unsubstituted saturated linear alkyl chain including 9 carbon atoms, p = n = 1 and q = 3,
as insecticide for treating the insects of the family of hemiptera, lepidoptera, diptera and/or coleoptera.

2. Use according to claim 1, for treating fruit, ornamental, cereal, vegetable and/or oleaginous crops.

3. Concentrate not including water including an effective quantity of an insecticide compound of formula (I) such as defined in claim 1 or of a mixture of insecticide compounds of formula (I), a surfactant and less than 20% by weight of a hydrocarbon aromatic solvent.

4. Concentrate including an effective quantity of an insecticide compound of formula (I) or of a mixture of insecticide compounds of formula (I) and a surfactant excluding salts of alkylarylsulfonic acid and alkylbiphenylsufonic acid.

5. Concentrate according to claim 4, wherein the surfactant is a biosurfactant.

6. Concentrate according to any of claims 3 to 5, including a second insecticide compound.

7. Concentrate according to any of claims 3 to 6, wherein the compound of formula (I) is 3-methylbutyl dodecanoate.

8. Solution insecticide including a concentrate according to any of claims 3 to 7 and water.

9. Method for preparing a solution according to claim 8, by emulsion, dispersion or dilution of the concentrate according to any of claims 3 to 7 in water.

10. Non-therapeutic use of a concentrate according to any of claims 3 to 7, or of the solution according to claim 8, as insecticide.

11. Method for controlling insects of the family of hemiptera, lepidoptera, diptera and/or coleoptera **characterised in that** a plant or a portion of the latter, that is to be treated, is put into contact with an effective dose of a compound of formula (I), of a mixture of compounds of formula (I), of a concentrate according to any of claims 3 to 7, or of the solution according to claim 8.

12. Method for controlling the insects of the family of hemiptera, lepidoptera, diptera and/or coleoptera **characterised in that** the ground that is to be treated is put into contact with an effective dose of a compound of formula (I), of a mixture of compounds of formula (I), of a concentrate according to any of claims 3 to 7, or of the solution according to claim 8.
